# EUROPEAN PATENT APPLICATION

(11) **EP 1 884 215 A1**
(43) Date of publication of application: **06.02.2008**
(21) Application number: 07252931.6
(22) Date of filing: 24.07.2007
(51) Int. Cl.: A61B 19/00, A61B 17/02

(54) **Apparatus for retracting patient tissue**

(30) Priority: 24.07.2006 US 459510
(71) Applicant: DePuy Products, Inc., Warsaw, IN 46581 (US)
(72) Inventor: Kuranda, Joseph, Mishawaka IN 46544 (US)
(74) Representative: Belcher, Simon James

(57) **Abstract**

A system for assisting a surgeon in performing an orthopaedic surgical procedure on a bone of a patient iuacludes a magnetic source configured to generate a magnetic field and a nonmagnetic surgical retractor. A controller is electrically coupled to a display device to control the display device to display indicia of a location of the bone of the patient based on the magnetic field while the nonmagnetic surgical retractor is located in the magnetic field.

## Description

The present disclosure relates to generally devices and methods for retracting tissue of a patient, and more particularly to devices and methods for retracting tissue of a patient during an orthopaedic surgical procedure.

Surgical retractors are devices used by surgeons and/or other healthcare providers during a surgical procedure to draw-back and restrain tissue of a patient. In particular, surgical retractors are used in minimally invasive orthopaedic procedures to increase the workspace volume of the minimally invasive surgical incision. Surgical retractors have a variety of sizes and shapes designed for particular purposes.

Because many surgical procedures, such as minimally invasive orthopaedic surgical procedures, generally restrict the surgeon's ability to see the operative area, surgeons are increasingly relying on computer systems, such as computer assisted orthopaedic surgery (CAOS) systems, to assist in the surgical operation. CAOS systems assist surgeons in the performance of orthopaedic surgical procedures by, for example, displaying images illustrating surgical steps of the surgical procedure being performed. In addition or alternatively, CAOS systems may provide surgical navigation to the surgeon by tracking and displaying indicia of the location of relevant bones of the patient, surgical tools used by the surgeon, and/or the like.

In one aspect, the invention provides a system for assisting a surgeon in performing an orthopaedic surgical procedure on a bone of a patient may include a display device, a magnetic source configured to generate a magnetic field, a nonmagnetic surgical retractor, and/or a controller electrically coupled to the display device. The controller may be configured to control the display device to display indicia of a location of the bone of the patient based on the magnetic field while the nonmagnetic surgical retractor is located in the magnetic field. The controller may also be configured to determine the indicia of the location of the bone based on the magnetic field while the nonmagnetic surgical retractor is positioned substantially in the magnetic field. The nonmagnetic surgical retractor may be formed from, for example, a polymeric material, or a shape memory alloy especially a nickel-titanium based alloy such as that known as nitinol.

The system can be used in a method for assisting a surgeon in performing an orthopaedic surgical procedure may include generating an incision in the tissue of a patient in the presence of a magnetic field. The magnetic field may be generated by a computer assisted orthopaedic surgery system. The method may also include retracting the tissue around the incision with a nonmagnetic surgical retractor in the presence of the magnetic field. The nonmagnetic surgical retractor may be formed from, for example, a polymeric material, or a shape memory alloy especially a nickel-titanium based alloy such as that known as nitinol. The method may also include displaying indicia of a location of the bone on the display device based on the magnetic field. In addition, the method may include altering the shape of the nonmagnetic surgical retractor. For example the shape of the nonmagnetic surgical retractor may be altered based on physical attributes of the patient. Additionally, the method may include positioning the nonmagnetic surgical retractor in the magnetic field and determining the indicia of the location of the bone while the nonmagnetic surgical retractor is positioned in the magnetic field.

The system can be used in a method for assisting a surgeon in performing an orthopaedic surgical procedure on a bone of a patient may include generating a magnetic field in the region of the bone of the patient and positioning a surgical retractor in the magnetic field. The surgical retractor may be a nonmagnetic surgical retractor and may be formed from, for example, a polymeric material, or a shape memory alloy especially a nickel-titanium based alloy such as that known as nitinol. The method may also include determining a location of the bone of the patient based on the magnetic field while the surgical retractor is positioned in the magnetic field.

Embodiments of the invention will now be described by way of example with reference to the accompanying drawings, in which:
FIG. 1 is a perspective view of a computer assisted orthopaedic surgery (CAOS) system;
FIG. 2 is a simplified diagram of the CAOS system of FIG. 1;
FIG. 3 is a simplified diagram of another embodiment of a CAOS system;
FIG. 4 is a simplified diagram of yet another embodiment of a CAOS system;
FIG. 5 is a simplified flowchart of an algorithm for assisting a surgeon in the performance of an orthopaedic surgical procedure; and
FIGS. 6 is an illustration of one embodiment of a nonmagnetic surgical retractor that may be used with the CAOS systems of FIGS. 3 and 4.

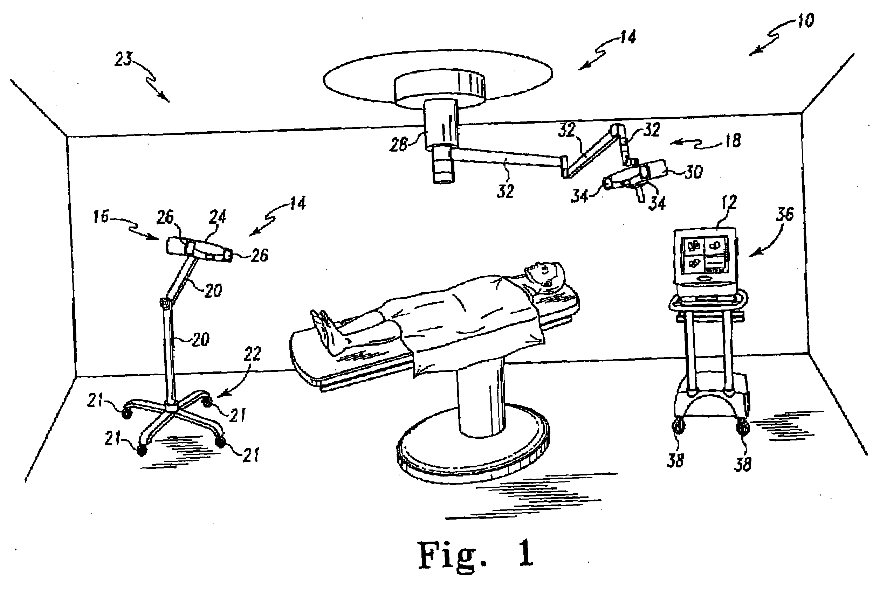

Referring to the drawings, FIG. 1 shows a computer assisted orthopaedic surgery (CAQS) system 10 includes a computer 12 and a camera unit 14. The CAOS system 10 may be embodied as any type of computer assisted orthopaedic surgery system. Preferably, the CAOS system 10 is embodied as one or more computer assisted orthopaedic surgery systems commercially available from DePuy Orthopaedics, Inc. of Warsaw, Indiana and/or one or more computer assisted orthopaedic surgery systems commercially available from BrainLAB of Heimstetten, Germany. The camera unit 14 may be embodied as a mobile camera unit 16 or a fixed camera unit 18. In some embodiments, the system 10 may include both types of camera units 16, 18. The mobile camera unit 16 includes a stand 20 coupled with a base 22. The base 22 may include a number of wheels 24 to allow the mobile camera unit 16 to be repositioned within a hospital room 23. The mobile camera unit 16 includes a camera head 24. The camera head 24 includes two cameras 26. The camera head 24 may be positioned relative to the stand 20 such that the field of view of the cameras 26 may be adjusted. The fixed camera unit 18 is similar to the mobile camera unit 16 and includes a base 28, a camera head 30, and an arm 32 coupling the camera head 28 with the base 28. In some embodiments, other peripherals, such as display screens, lights, and the like, may also be coupled with the base 28. The camera head 30 includes two cameras 34. The fixed camera unit 18 may be coupled to a ceiling, as shown in FIG. 1, or a wall of the hospital room. Similar to the camera head 24 of the camera unit 16, the camera head 30 may be positioned relative to the arm 32 such that the field of view of the cameras 34 may be adjusted. The camera units 14,16, 18 are communicatively coupled with the computer 12. The computer 12 may be mounted on or otherwise coupled with a cart 36 having a number of wheels 38 to allow the computer 12 to be positioned near the surgeon during the performance of the orthopaedic surgical procedure.

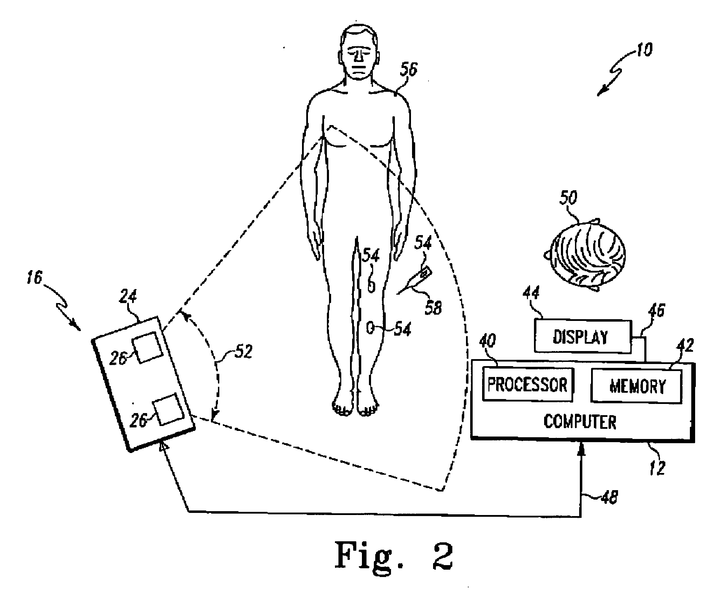

FIG. 2 shows the computer 12 with a processor 40 and a memory device 42. The processor 40 may be embodied as any type of processor including, for example, discrete processing circuitry (e.g., a collection of logic devices), general purpose integrated circuit(s), and/or application specific integrated circuit(s) (i.e., ASICs). The memory device 42 may be embodied as any type of memory device and may include one or more memory types, such as, random access memory (i.e., RAM) and/or read-only memory (i.e., ROM). In addition, the computer 12 may include other devices and circuitry typically found in a computer for performing the functions described herein such as, for example, a hard drive, input/output circuitry, and the like.

The computer 12 is communicatively coupled with a display device 44 by means of a communication link 46. Although shown in FIG. 2 as separate from the computer 12, the display device 44 may form a portion of the computer 12 in some embodiments. Additionally, in some embodiments, the display device 44 or an additional display device may be positioned away from the computer 12. For example, the display device 44 may be coupled with the ceiling or wall of the operating room wherein the orthopaedic surgical procedure is to be performed. Additionally or alternatively, the display device 44 may be embodied as a virtual display such as a holographic display, a body mounted display such as a heads-up display, or the like. The computer 12 may also be coupled with a number of input devices such as a keyboard and/or a mouse for providing data input to the computer 12. However, as shown in the drawings, the display device 44 is a touch-screen display device capable of receiving inputs from an orthopaedic surgeon 50. That is, the surgeon 50 can provide input data to the computer I2, such as making a selection from a number of on-screen choices, by simply touching the screen of the display device 44.

The computer 12 is also communicatively coupled with the camera, unit 16 (and/or 18) by means of a communication link 48. Preferably, the communication link 48 is a wired communication link but, in some embodiments, may be embodied as a wireless communication link. In embodiments wherein the communication link 48 is a wireless signal path, the camera unit 16 and the computer 12 include wireless transceivers such that the computer 12 and camera unit 16 can transmit and receive data (e.g., image data). Although only the mobile camera unit 16 is shown in FIG. 2, it should be appreciated that the fixed camera unit 18 may alternatively be used or may be used in addition to the mobile camera unit 16.

The CAOS system 10 of FIGS. 1 and 2 utilizes infxa-red tracking to determine the location of relevant bones of a patient 56 and/or orthopaedic surgical tools 58. As such, the CAOS system 10 includes a number of sensors or sensor arrays 54 which may be coupled to the relevant bones of the patient 56 and/or with the orthopaedic surgical tools 58. Each of the sensors 54 includes a number of reflective elements or sensors. The reflective elements are embodied as spheres in the described embodiment, but may have other geometric shapes in other embodiments. The reflective elements of the sensors 54 are positioned in a predefined configuration that that allows the computer 12 to determine the identity of the bone of the patient 56 or the orthopaedic surgical tool 58 to which the particular sensor 54 is coupled. That is, when the sensor 54 is positioned in a field of view 52 of the camera head 24, as shown in FIG. 2, the computer 12 is configured to determine the identity of the bone or orthopaedic surgical tool 58 based on the images received from the camera head 24. Additionally, based on the relative position of the reflective elements of the sensor 54, the computer 12 is configured to determine the location and orientation of the bone of the patient 56 and/or orthopaedic surgical tool 58

The CAOS system 10 may be used by the orthopaedic surgeon 50 to assist in any type of orthopaedic surgical procedure including, for example, a total knee replacement procedure. To do so, the computer 12 and/or the display device 44 are positioned within the view of the surgeon 50. As discussed above, the computer 12 may be coupled with a movable cart 36 to facilitate such positioning. The camera unit 16 (and/or camera unit 18) is positioned such that the field of view 52 of the camera head 24 covers the portion of the patient 56 upon which the orthopaedic surgical procedure is to be performed, as shown in FIG. 2.

During the performance of the orthopaedic surgical procedure, the computer 12 of the CAOS system 10 is programmed or otherwise configured to display images of the individual surgical procedure steps which form the orthopaedic surgical procedure being performed. The images may be graphically rendered images or graphically enhanced photographic images. For example, the images may include three dimensional rendered images of the relevant anatomical portions of a patient. The surgeon 50 may interact with the computer 12 to display the images of the various surgical steps in sequential order. In addition, the surgeon may interact with the computer 12 to view previously displayed images of surgical steps, selectively view images, instruct the computer 12 to render the anatomical result of a proposed surgical step or procedure, or perform other surgical related functions. For example, the surgeon may view rendered images of the resulting bone structure of different bone resection procedures. In this way, the CAOS system 10 provides a surgical "walk-through" for the surgeon 50 to follow while performing the orthopaedic surgical procedure.

In some embodiments, the surgeon 50 may also interact with the computer 12 to control various devices of the system 10. For example, the surgeon 50 may interact with the system 10 to control user preferences or settings of the display device 44. Further, the computer 12 may prompt the surgeon 50 for responses, For example, the computer 12 may prompt the surgeon to inquire if the surgeon has completed the current surgical step, if the surgeon would like to view other images, and the like.

The camera unit 16 and the computer 12 also cooperate to provide the surgeon with navigational data during the orthopaedic surgical procedure. That is, the computer 12 determines and displays the location of the relevant bones and the surgical tools 58 based on the data (e.g., images) received from the camera head 24 by means of the communication link 48. To do so, the computer 12 compares the image data received from each of the cameras 26 and determines the location and orientation of the bones and tools 58 based on the relative location and orientation of the sensor arrays 54. The navigational data displayed to the surgeon 50 is continually updated. In this way, the CAOS system 10 provides visual feedback of the locations of relevant bones and surgical tools for the surgeon 50 to monitor while performing the orthopaedic surgical procedure.

Although the CAOS system 10 described above in regard to FIGS. 1 and 2 utilizes an infra-red tracking technology to determine the location of the relevant bones of the patient 56 and the orthopaedic surgical tools 58, other tracking methodologies and technologies may be used in other embodiments. For example, in some embodiments, any number of magnetic fields may be used to determine the location of the relevant bones and/or orthopaedic surgical tools 58. As used herein, the term "magnetic field" is intended to refer to any magnetic and/or electromagnetic field generated by any number of permanent magnets and/or electromagnets.

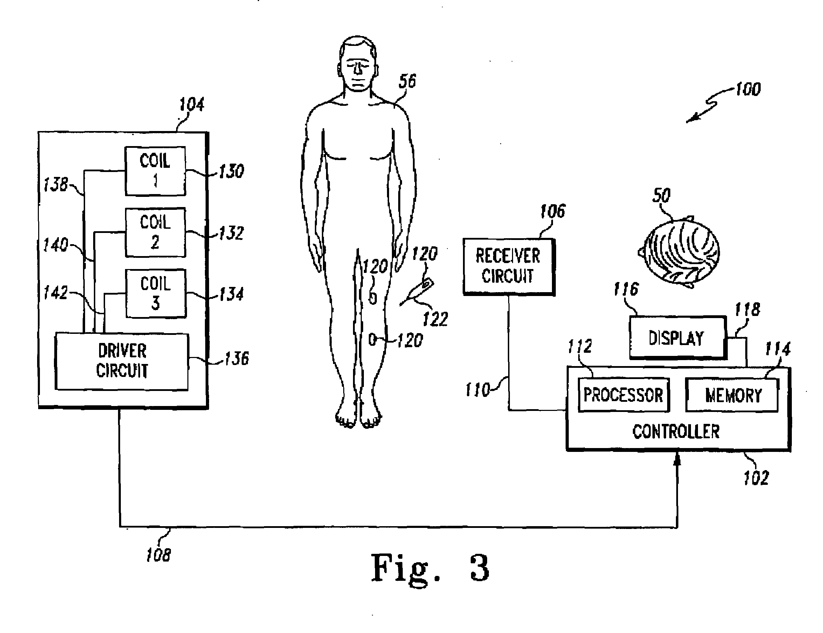

For example, in one embodiment, a CAOS system 100 includes a controller 102, a coil array 104, and a receiver circuit 106 as shown in FIG. 3. The controller 102 is communicatively coupled to the coil array 104 by means of a number of communication links 108 and to the receiver circuit 106 by means of a number of communication links 110. The communication links 108, 110 may be embodied as any type of communication links capable of facilitating electrical communication between the controller 102 and the coil array 104 and the receiver circuit 110, respectively. For example, the communication links 108, 110 may be embodied as any number of wires, cables, or the like.

The controller 102 includes a processor 112 and a memory device 114. The processor 112 may be embodied as any type of processor including, for example, discrete processing circuitry (e.g., a collection of logic devices), general purpose integrated circuit(s), and/or application specific integrated circuit(s) (i.e., ASICs). The memory device 114 may be embodied as any type of memory device and may include one or more memory types, such as, random access memory (i.e., RAM) and/or read-only memory (i.e., ROM). In addition, the controller 102 may include other devices and circuitry typically found in a computer for performing the functions described herein such as, for example, a hard drive, input/output circuitry, and the like.

The controller 102 is communicatively coupled with a display device 116 by means of a communication link 118. Although shown in FIG. 3 as separate from the computer 102, the display device 116 may form a portion of the controller 102 in some embodiments. Additionally, in some embodiments, the display device 116 or an additional display device may be positioned away from the controller 102. For example, the display device 116 may be coupled to the ceiling or wall of the operating room wherein the orthopaedic surgical procedure is to be performed. Additionally or alternatively, the display device 116 may be embodied as a virtual display such as a holographic display, a body mounted display such as a heads-up display, or the like. The controller 102 may also be coupled with a number of input devices such as a keyboard and/or a mouse for providing data input to the controller 102. However, in the described embodiment, the display device 116 is a touch-screen display device capable of receiving inputs from the orthopaedic surgeon 50 similar to the display device 44 described above in regard to FIG. 2. That is, the surgeon 50 can provide input data to the controller 102, such as making a selection from a number of on-screen choices, by simply touching the screen of the display device 116.

The coil array 104 includes a number of coils 130,132,134 and a coil driver circuit 136. Although only three coils 130, 132, 134 are shown in FIG. 3, in other embodiments, additional coils may be used. The coil driver circuit 136 is coupled to the coils 130, 132, 134 by means of communication links 138, 140, 142, respectively. The communication links 138, 140, 142 may be embodied as any type of communication link capable of facilitating electrical communication between the coils 130, 132, 134 and the driver circuit 136. For example, the communication links may be embodied as any number of wires, cables, printed circuit board traces, and/or the like.

The system 100 may also include a number of magnetic sensors 120. Similar to the sensor arrays 54, the magnetic sensors 120 may be coupled to a bone of the patient 56 and/or a number of orthopaedic surgical tools 122 such as a registration pointer, bone saw, or the like. The magnetic sensors 120 also include processing circuitry and wireless transmitter circuitry configured to communicate with the receiver circuit 106 by means of a wireless communication.

In operation, the driver circuit 136 is configured to energize the coils 130, 132, 134 by means of a number of activation signals of varying frequencies supplied on the communication links 138,140,142, respectively. The driver circuit 136 generates the activation signals in response to a control signal received from the controller 102 on the communication link 108. In response to the activation signals, each coil 130, 132, 134 generates an electromagnetic field having different, respective sets of frequencies. The magnetic sensors 120 are positioned in the electromagnetic field generated by the coils 130, 132, 134. Each of the magnetic sensors 120 include a number of sensor coils in which a current is generated in response to each magnetic field. The processing circuitry of each magnetic sensor 120 is configured to determine location data indicative of the location of the magnetic sensor relative to the coils 130,132, 134 based on the currents induced in the sensor coils. For example, the processing circuitry may be configured to determine the location data based on the amplitude of the induced currents. Once the location data is determined, the transmitter circuitry of each magnetic sensor 120 transmits the location data to the receiver circuit 106. The controller 102 receives the location data from the receiver circuit 106 by means of the communication link 110 and is configured to display indicia of the location of the magnetic sensors 120 and/or the bones, orthopaedic medical devices and tools, and the like to which the magnetic sensors 120 are coupled. As such, in the embodiment shown in FIG. 3, magnetic/electromagnetic fields are used to determine the location of relevant bones of the patient 56 and/or orthopaedic surgical tools 122 and provide an amount of surgical navigation for the surgeon 50. Similar systems and methods for determining the location of patient's bones, sensors, and/or orthopaedic surgical tools based on signals received by magnetic and/or electromagnetic sensors are disclosed in WO-2005/086062 and WO-2005/087125.

In another embodiment, a CAOS system 200 includes a controller 202, and a magnetic sensor array 204 as shown in FIG. 3. The controller 202 is communicatively coupled to the magnetic sensor array 204 by means of a number of communication links 208. The communication links 208 may be embodied as any type of a wireless and/or wired communication links capable of facilitating electrical communication between the controller 102 and the magnetic sensor array 204. For example, the communication links 208 may be embodied as any number of wires, cables, or the like.

Similar to the controller 102 of the system 100, the controller 202 includes a processor 212 and a memory device 214. The processor 212 may be embodied as any type of processor including, for example, discrete processing circuitry (e.g., a collection of logic devices), general purpose integrated circuit(s), and/or application specific integrated circuit(s) (i.e., ASICs). The memory device 214 may be embodied as any type of memory device and may include one or more memory types, such as, random access memory (i.e., RAM) and/or read-only memory (i,e., ROM). In addition, the controller 202 may include other devices and circuitry typically found in a computer for performing the functions described herein such as, for example, a hard drive, input/output circuitry, and the like.

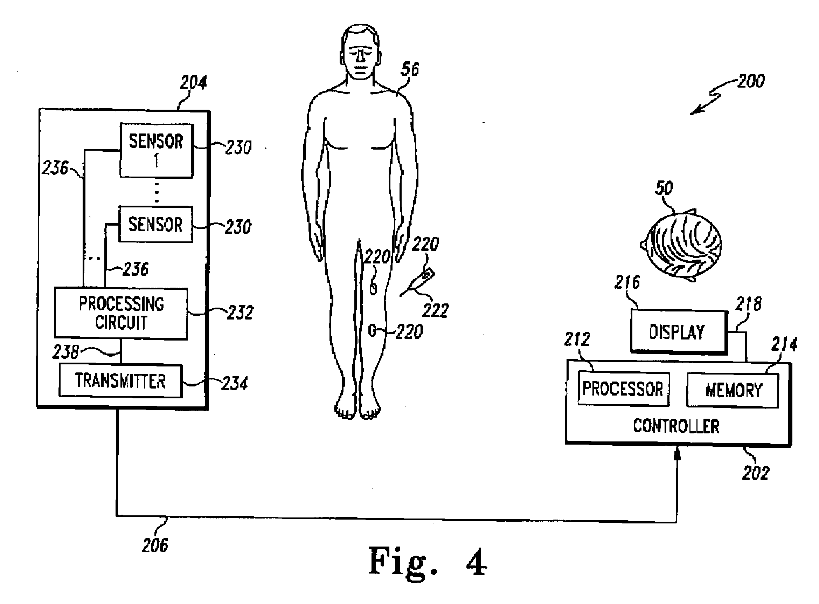

The controller 202 is also communicatively coupled with a display device 216 by means of a communication link 218. Although shown in FIG. 4 as separate from the computer 202, the display device 216 may form a portion of the controller 202 in some embodiments. Additionally, in some embodiments, the display device 216 or an additional display device may be positioned away from the controller 202. For example, the display device 216 may be coupled to the ceiling or wall of the operating room wherein the orthopaedic surgical procedure is to be performed. Additionally or alternatively, the display device 1216 may be embodied as a virtual display such as a holographic display, a body mounted display such as a heads-up display, or the like. The controller 202 may also be coupled with a number of input devices such as a keyboard and/or a mouse for providing data input to the controller 202. However, similar to the system 100, in the embodiment shown in FIG. 4, the display device 216 is a touch-screen display device capable of receiving inputs from the orthopaedic surgeon 50 similar to the display device 44 described above in regard to FIG. 2.

The magnetic sensor array 204 includes a number of magnetic or electromagnetic sensors 230, a processing circuit 232, and a transmitter 234. The processing circuit 232 is communicatively coupled to the sensors 230 by means of a number of communication links 236 and to the transmitter 234 by means of a number of communication links 238. The communication links 236, 238 may be embodied as any type of communication links capable of facilitating electrical communication between the processing circuit 232 and the sensors 230 and between the processing circuit 232 and the transmitter 234, respectively. For example, the communication links 236, 238 may be embodied as any number of wires, cables, printed circuit board traces, and/or the like. The magnetic sensor array 204 may be embodied as a handheld device in some embodiments. Additionally or alternatively, the magnetic sensor array 204 may be embodied as a mounted device secured to, for example, an operating table, a ceiling, a floor, a movable cart or assembly, and/or the like. Additionally, although only a single magnetic sensor array 204 is shown in FIG. 4, in other embodiments, the system 200 may include any number of magnetic sensor arrays 204.

The system 100 may also include a number of magnetic sources 120. Similar to the sensor arrays 54, the magnetic sensors 220 may be coupled to a bone of the patient 56 and/or to any number of orthopaedic surgical tools 222 such as a registration pointer, ligament balancer, bone saw, or the like. The magnetic sources 220 may be embodied as, for example, permanent magnets. Alternatively, in some embodiments, the magnetic sources 120 may be embodied as electromagnets- Regardless, the magnetic sources 120 are configured to generate a magnetic/electromagnetic field.

In operation, the magnetic sensor array 204 is positioned in the magnetic fields generated by the magnetic sources 120. The distance from which the magnetic sensor array 204 is positioned relative to the respective magnetic source 120 may be determined based on the type and implementation of magnetic source 120. For example, in some embodiments, the magnetic sources 120 may be coupled to the bone or bones of the patient 56 pzc-operatively. In such embodiments, the magnetic sensor array 204 may be positioned close to the skin of the patient 56. Once properly positioned in the magnetic field of the magnetic sources 220, the sensors 230 produce a data signal on the respective communication link 236 based on the magnetic field. The sensors 230 are positioned in a configuration in the magnetic sensor array 204 such that each sensor produces a data signal based on the relevant magnetic source 220. The processing circuit 232 receives each of the data signals produced by the sensors 230 and determines location data indicative of the location and/or position of the relevant magnetic source with respect to the magnetic sensor array 204 based on the combination of data signals. The transmitter 234 transmits the location data from the magnetic sensor array 204 to the controller 202 by means of the communication link 206. The controller 202 is configured to display indicia the location and/or position of the magnetic sources 220, the relevant bones of the patient 56, and/or orthopaedic surgical tools 222. As such, in the embodiment shown in FIG. 4, magnetic/electromagnetic fields are used to determine the location of relevant bones of the patient 56 and/or orthopaedic surgical tools and provide an amount of surgical navigation for the surgeon 50. Similar systems and methods for determining the location of patient's bones, sensors, and/or orthopaedic surgical tools based on signals received by magnetic and/or electromagnetic sensors are disclosed in EP-A-1803394, EP-A-1803412, EP-A-1803413 and EP-A-1803414.

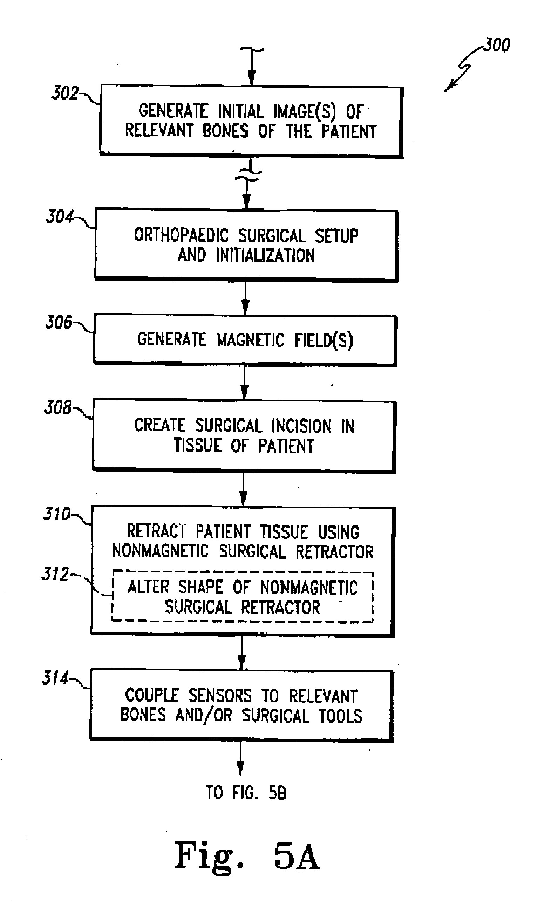

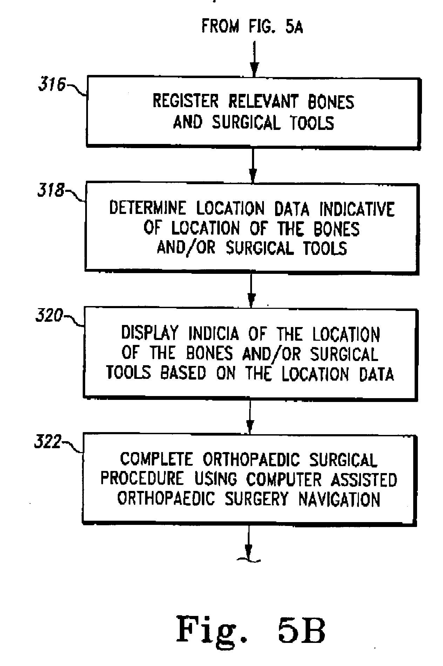

Referring now to FIG. 5, an algorithm 300 for assisting a surgeon in performing an orthopaedic surgical procedure begins with a process step 302 in which medical images of a relevant bone or bones of a patient are generated. Typically, the medical images are generated pre-operatively in preparation for an orthopaedic surgical procedure. The medical images may include any number of medical images and may be embodied as any type of image capable of providing indicia of the relevant bone or bones. For example, the medical images may be embodied as any number of X-ray images, magnetic resonance imaging (MRI) images, computerized tomography (CT) images, or the like. As such, any number of imaging devices and systems, such as an X-ray machine, a CT scanner, and/or an MRI machine may be used to generate the medical images. In some embodiments, such as those embodiments wherein the medical images are generated pre-operatively, the medical images may be stored in a storage device such as a database for later retrieval.

Once the initial images of the relevant bones of the patient have been generated in process step 302, an orthopaedic surgery setup and initialization procedure is performed in process step 304. The orthopaedic surgery setup may include any pre-operative steps and/or procedures necessary for the preparation of performing the orthopaedic surgical procedure. For example, the surgery setup may include a surgical planning procedure, analysis of medical images, surgical pre-operation procedures, and/or the like. The initialization procedure may include any steps required to prepare the CAOS system 100, 200 for use in an orthopaedic surgical procedure. For example, the initialization procedure may include selection of user preferences on the controller 102, 202, a communication verification procedure to ensure the devices of the CAOS system 100, 200 are properly communicating with each other and/or the relevant controller 102, 302, any other initial setup procedures for the controller 102, 302, and/or the like.

Once the orthopaedic surgical setup and initialization procedures have been performed in the process step 304, one or more magnetic fields are generated in process step 306. For example, in embodiments similar to the system 100 described above in regard to FIG. 3, the magnetic field(s) may be generated or produced by a number of electromagnetic coils (e.g., the coils 130, 132, 134). Alternatively, in embodiments similar to the system 200 described above in regard to FIG. 4, the magnetic field(s) may be generated or produced by a number of magnetic sources, such as permanent magnets (e.g., magnetic sources 220).

Subsequently, in process step 308, the surgeon 50 creates a surgical incision in a tissue of the patient 56 according to the particular orthopaedic surgical procedure being performed. For example, in a total-knee arthoplasty (TKA) orthopaedic surgical procedure, the surgeon 50 may create an incision in the tissue of the patient in the region of the relevant knee of the patient 56. In some embodiments, the incision is a minimally invasive incision. It should be appreciated that although only a single surgical incision step 306 is referred to in the algorithm 300, any number of surgical incision steps may be included in other embodiments based on, for example, the particular orthopaedic surgical procedure being performed.

Once the surgeon 50 has created the surgical incision in step 308, the patient tissue surrounding the surgical incision is retracted in process step 310. To do so, the surgeon 50 tetracts the patient tissue with one or more nonmagnetic surgical retractors in the presence of the magnetic field(s) generated in process step 306. Because the retractor(s) are nonmagnetic, the introduction of the nonmagnetic retractor into the magnetic field(s) does not substantially interfere with the operation of the magnetic/electromagnetic sensors 120, 230. That is, the systems 100, 200 are capable of determining location data, displaying indicia of the location of relevant bones of the patient 56 and/or orthopaedic surgical tools 122, 222, and provide surgical navigation to the surgeon 50 based on the magnetic fields generated by the coil array 104 and magnetic sources 220, respectively, while the nonmagnetic surgical retractor is within the generated magnetic fields.

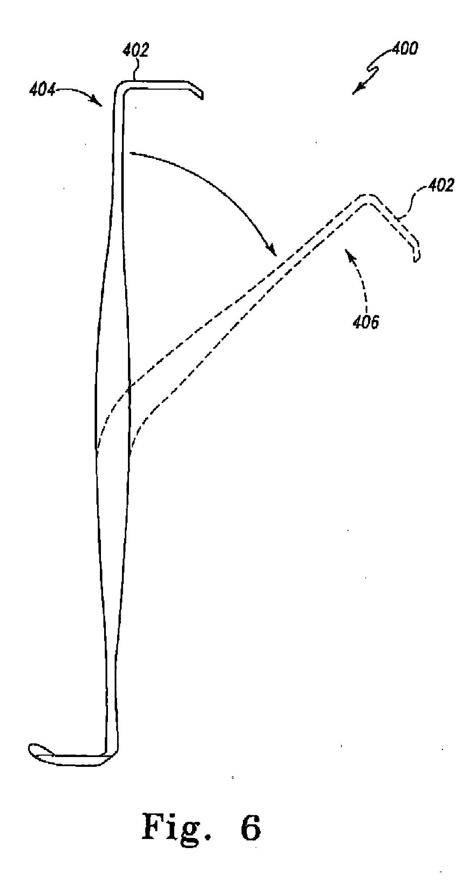

The nonmagnetic surgical retractor may be embodied as any type of surgical retractor commonly used in an orthopaedic surgical procedure. As such, the nonmagnetic surgical retractor may be of any shape, size, and configuration as required for the particular orthopaedic surgical procedure being performed. For example, the nonmagnetic surgical retractor may be embodied as a Chandler retractor, a collateral ligament retractor, an Engh intracondylar notch retractor, a Lipscomb retractor, a PCL retractor, an Army-Navy retractor, a patellar retractor, an s-shaped retractor, a Z retractor, or any other type of retractor commonly used in an orthopaedic surgical procedure formed from a nonmagnetic material. One exemplary nonmagnetic surgical retractor 400 is shown in FIG. 6. The surgical retractor 400 may be formed from any material that is not capable of being substantially magnetized. For example, in one embodiment, the retractor 400 is formed from a plastic material. In another embodiment, the retractor 400 is formed from a nonmagnetic shape memory alloy material such as, for example, a nickel-titanium based (e.g., a Nitinol alloy). However, in yet other embodiments, the retractor 400 may be formed from any type of other nonmagnetic material.

In embodiments, wherein the retractor 400 is formed from a shape memory alloy the process step 310 may include a sub-step 312 in which the shape of the retractor 400 is altered. The shape of the retractor 400 may be altered by bending, twisting, or otherwise deforming any portion of the retractor 400 based on any physical attribute of the patient 50 such as the size and/or location of the incision, the weight of the patient, the shape and/or size of the patient's bony anatomy, and/or the like. For example, as shown in FIG. 6, the surgeon 50 may alter the shape of the retractor 400 by bending a distal end 402 of the retractor 400 from a first position 404 to a second position 406. Because the nonmagnetic surgical retractor 400 is formed from a shape memory alloy, the retractor 400 retains the shape of the second position 406.

Once the tissue of the patient has been retracted in process step 310, the magnetic sensors 120 or the magnetic sources 220 are coupled to the relevant bones of the patient 56 and/or the orthopaedic surgical devices 122, 222 in process step 314. Next, in process step 316, the relevant bones and/or orthopaedic surgical devices 122, 222 are registered with the controller 102, 202. The registration process may include, for example, contacting a registration pointer to various points of the relevant bones of a patient, for example as disclosed in EP-A-1803394, EP-A-1803412, EP-A-1803413 and EP-A-1803414.

Once the relevant bones of the patient 56 and/or orthopaedic surgical devices 122, 222 have been registered in process step 316, the controller 102, 202 determines location data indicative of the location of the relevant bones and/or devices 122, 222. For example, the controller 102 determines the location data based on the data signals received from the magnetic sensors 120 by the receiver circuit 106. Similarly, the controller 202 determines the location data based on data signals received from the magnetic sensor array 204. Regardless, it should be appreciated that the location data is determined based on signals produced by one or more magnetic fields while the nonmagnetic surgical retractor is present in the one or more magnetic fields.

Once the location data has been determined, indicia of the location of the relevant bones and/or orthopaedic surgical devices 122, 222 is displayed to the surgeon 50 on the display 116, 216 in process step 320. The surgeon 50 completes the orthopaedic surgical procedure in process step 322 by use of the surgical navigation provided by the indicia of the location of the relevant bones and/or orthopaedic surgical devices 122,222 displayed on the display 116, 216. As such, it should be appreciated that during the remaining steps of the orthopaedic surgical navigation, the process steps 318 and 320 may be repeated. That is, location data may be continuously, continually, or periodically determined based on the data signals received from the coil array 104 or the magnetic sensor array 204 and the indicia of the location of the bones and/or orthopaedic surgical devices 122, 222 may be updated on the display 116, 216.

## Claims

1. A system for assisting a surgeon in performing an orthopaedic surgical procedure on a bone of a patient, the system comprising:
a display device;
a magnetic source configured to generate a magnetic field;
a nonmagnetic surgical retractor; and
a controller electrically coupled to the display device and configured to control the display device to display indicia of a location of the bone of the patient based on the magnetic field while the nonmagnetic surgical retractor is located in the magnetic field.

2. The system of claim 1, wherein the nonmagnetic surgical retractor is formed from a plastic material.

3. The system of claim 1, wherein the nonmagnetic surgical retractor is formed from a nickel-titanium alloy.

4. The system of claim 3 , wherein the nonmagnetic surgical retractor is formed from a shape memory alloy.

5. The system of claim 1, wherein controller is configured to determine the indicia of the location of the bone based on the magnetic field while the nonmagnetic surgical retractor is positioned substantially in the magnetic field,
